# DEMANDE DE BREVET EUROPEEN

(11) **EP 3 777 792 A1**
(43) Date de publication de la demande: **17.02.2021**
(21) Numéro de dépôt: 20190532.0
(22) Date de dépôt: 11.08.2020
(51) Int. Cl.: A61F 11/08

(54) **ENSEMBLE POUR FABRIQUER AU MOINS UN DISPOSITIF D'OBTURATION D'UN CONDUIT AUDITIF ET PROCÉDÉ DE FABRICATION D'AU MOINS UN DISPOSITIF D'OBTURATION D'UN CONDUIT AUDITIF**

(30) Priorité: 13.08.2019 FR 1909192
(71) Demandeur: De Travieso, Harold Gonzague Bruno, 1250-244 Lisbonne (PT); Jean, Simon Henri, 1070-219 Lisbonne (PT); Benard, Fabrice, 1050-218 Lisbonne (PT)
(72) Inventeur: De Travieso, Harold Gonzague Bruno, 1250-244 Lisbonne (PT); Jean, Simon Henri, 1070-219 Lisbonne (PT); Benard, Fabrice, 1050-218 Lisbonne (PT)
(74) Mandataire: Jacobacci Coralis Harle

(57) **Abrégé**

L'invention concerne un ensemble (1) pour fabriquer au moins un dispositif (2) d'obturation d'un conduit auditif (3,4).

Selon l'invention, l'ensemble (1) comprend un premier et un deuxième composant (5,6) comprenant chacun de l'huile de silicone vinylique, du dioxyde de silicium et un agent de démoulage, les deux composants (5,6) étant destinés à être mélangés ensemble pour former un troisième composant (7) homogène ayant une dureté comprise entre 10 et 30 shore A, et ledit troisième composant (7) est destiné à être introduit dans au moins un conduit auditif (3,4).

L'invention concerne également un procédé (25) de fabrication d'au moins un dispositif (2) d'obturation d'un conduit auditif (3,4).

Selon l'invention, le procédé (25) comprend :
- la mise en œuvre d'un premier composant (5) comprenant de l'huile de silicone vinylique, du dioxyde de silicium et un agent de démoulage,
- la mise en œuvre d'un deuxième composant (6) comprenant de l'huile de silicone, du dioxyde de silicium et un agent de démoulage,
- le malaxage des premier et deuxième composants (5,6) jusqu'à obtenir un troisième composant (7) homogène,
- l'introduction du troisième composant (7) dans un conduit auditif (3,4), et
- l'attente d'au moins trois minutes avant de le retirer.

## Description

La présente invention concerne le domaine technique des ensembles pour fabriquer au moins un dispositif d'obturation d'un conduit auditif. L'invention concerne également un procédé de fabrication d'au moins un dispositif d'obturation d'un conduit auditif.

Dans le domaine ci-dessus, sont connus des dispositifs d'obturation de conduit auditif à base de silicone, aussi appelés bouchon d'oreille, qui sont vendus préformés prêts à l'emploi pour une utilisation destinée à protéger du bruit, du vent et de l'eau lors d'activités aquatiques. Cependant de tels dispositifs ne sont pas adaptables à tous les conduits auditifs et peuvent donc induire des désagréments si le dispositif n'obstrue pas correctement le conduit auditif ou ne possède pas une forme adaptée à ce dernier. L'efficacité contre les bruits gênants ou contre l'introduction d'eau dans le conduit auditif peut être alors altérée. De plus, les dispositifs connus se dégradent rapidement et ne peuvent pas être utilisés sur le long terme.

Afin de remédier à ces inconvénients, il existe des bouchons d'oreille sur-mesure. De tels dispositifs nécessitent une prise d'empreintes des conduits auditifs de l'utilisateur chez un professionnel qui fabriquera ensuite un bouchon pour chaque oreille. Cela induit donc un certain temps d'attente pour obtenir le dispositif dont le coût d'achat est par ailleurs élevé.

Il est donc apparu le besoin de proposer une alternative aux dispositifs selon l'art antérieur.

A cet effet, l'invention concerne un ensemble pour fabriquer au moins un dispositif d'obturation d'un conduit auditif, l'ensemble comprenant un premier et un deuxième composant comprenant chacun de l'huile de silicone vinylique, du dioxyde de silicium et un agent de démoulage, les deux composants étant destinés à être mélangés ensemble pour former un troisième composant homogène ayant une dureté comprise entre 10 et 30 shore A. Le troisième composant obtenu est destiné à être introduit, avant polymérisation ou prise, dans au moins un conduit auditif. Ainsi, l'utilisateur façonne le troisième composant et procède lui-même à son introduction dans son ou ses conduits auditifs, l'empreinte obtenue forme alors un dispositif d'obturation pour conduit auditif ou bouchon d'oreille sur mesure. De plus, la mise en œuvre d'un ensemble comprenant deux composants permet d'en faciliter la conservation, puisque les deux composants ne sont pas en contact l'un avec l'autre évitant ainsi toute polymérisation ou durcissement.

Selon l'indice Shore A qui mesure la dureté des élastomères dits mous, notamment des silicones, plus le Shore est élevé, plus la matière est dure. Un bouchon d'oreille avec une dureté s'approchant de 10 Shore A sera relativement mou et offrira un certain confort pour dormir. Un bouchon d'oreille avec une dureté s'approchant de 30 Shore A sera plus dur mais sera plus efficace pour obturer les conduits auditifs dans des conditions plus extrêmes, telles qu'un vent fort ou une immersion dans l'eau de mer. Un bouchon d'oreille avec une dureté s'approchant de 20 Shore A offrira un confort optimal pour de nombreuses activités et notamment d'activités aquatiques telles que la natation ou le surf.

Selon une caractéristique de l'invention, le premier composant comprend en outre un catalyseur au platine et le deuxième composant comprend en outre un agent de réticulation d'huile de silicone contenant de l'hydrogène et un agent de contrôle de la structure. L'ajout de ces constituants différents pour les deux composants permet, d'une part, de favoriser l'homogénéité du troisième composant obtenu après mélange, d'autre part, d'obtenir un troisième composant ayant un aspect de surface lissée, et, par ailleurs, de réduire le temps de durcissement ou polymérisation permettant ainsi d'obtenir rapidement un moulage du conduit auditif.

Selon une autre caractéristique de l'invention, le premier composant comprend de l'huile de silicone vinylique dans une proportion en masse comprise entre 35 et 65 %, de préférence entre 43 et 57%, du dioxyde de silicium dans une proportion en masse comprise entre 25 et 50 %, de préférence entre 30 et 44%, un catalyseur au platine dans une proportion en masse de moins de 3 %, de préférence de moins de 1% et un agent de démoulage dans une proportion en masse d'environ 12% (typiquement 5-20 %, de préférence 8-15 %), et/ou le deuxième composant comprend de l'huile de silicone vinylique dans une proportion en masse d'environ 45% (typiquement 35-55 %, de préférence 40-50 %), du dioxyde de silicium dans une proportion en masse d'environ 42% (typiquement 35-55 %, de préférence 37-47 %), un agent de réticulation d'huile de silicone contenant de l'hydrogène dans une proportion en masse d'environ 1% (typiquement 0,2-5 %, de préférence 0,5-3 %), un agent de contrôle de la structure et un agent de démoulage dans une proportion en masse d'environ 12% (typiquement 5-20 %, de préférence 8-15 %). Ces différents rapports de pourcentage sont préférés et non limitatifs. Ils permettent, d'une part, de favoriser l'obtention du troisième composant homogène ayant une dureté comprise entre 10 et 30 shore A, et d'autre part, d'obtenir un troisième composant qui garde ses propriétés physiques plus longtemps et donc qui ne se dégrade pas rapidement.

Selon encore une autre caractéristique de l'invention, le premier composant comprend de l'huile de silicone vinylique dans une proportion en masse d'environ 51% et du dioxyde de silicium dans une proportion en masse d'environ 36%. Ces proportions données en masse permettent d'obtenir un troisième composant ayant une dureté de sensiblement 20 shore A qui est idéale pour le confort de l'utilisateur, notamment pour les activités aquatiques.

Le dioxyde de silicium (silice) présent dans le premier composant et/ou le second composant peut être de différentes natures, amorphe ou cristalline, telles que, sans limitation, une silice précipitée, un gel de silice, une silice à l'arc, une silice pyrogénée, une fumée de silice (microsilice), un sol de silice (silice colloïdale), une terre de diatomée, une silice cristalline telle que le quartz, la cristobalite ou la tridymite, ou une silice microcristalline telle que la calcédoine. Plusieurs types de silices peuvent être combinés.

Selon encore une autre caractéristique de l'invention, le dioxyde de silicium présent dans le premier composant se trouve au moins partiellement sous la forme de silice pyrogénée, préférentiellement une silice pyrogénée de caractère hydrophile, amorphe. Elle joue un rôle d'agent épaississant et thixotrope et également d'agent de renforcement et antiagglomérant. Elle rend l'huile de silicone vinylique pâteuse. Selon un mode de réalisation, le premier composant comprend de 0,5 à 5 % en masse de silice pyrogénée, de préférence de 1 à 3 %, typiquement 1,7 %, par rapport à la masse du premier composant. Un exemple non limitatif d'un tel composé est la silice (SiO₂) pyrogénée correspondant au numéro CAS 112945-52-5, de surface spécifique typiquement de 200 ± 25 m²/g. Selon un autre mode de réalisation, la silice pyrogénée représente au moins 5 %, au moins 10 %, au moins 20 %, au moins 50 %, au moins 75 % ou 100 % en masse du dioxyde de silicium employé dans le premier composant.

Selon encore une autre caractéristique de l'invention, le dioxyde de silicium présent dans le second composant se trouve au moins partiellement sous la forme de silice pyrogénée, préférentiellement une silice pyrogénée de caractère hydrophile, amorphe. Elle peut être utilisée dans les mêmes gammes de proportions que dans le cas du premier composant, et un exemple non limitatif d'un tel composé est la silice (SiO₂) pyrogénée correspondant au numéro CAS 112945-52-5, de surface spécifique typiquement de 200 ± 25 m²/g.

Selon encore une autre caractéristique de l'invention, le dioxyde de silicium présent dans le premier et/ou le second composant se trouve au moins partiellement sous la forme de silice cristalline, de préférence finement divisée. Un exemple non limitatif d'un tel composé est le quartz correspondant au numéro CAS 14808-60-7, de surface spécifique typiquement de 125 ± 20 m²/g. Dans un mode de réalisation, le premier et/ou le second composant contient au moins 50 %, au moins 75 %, au moins 90 %, au moins 94 %, au moins 95 % ou de 90 % à 96 % en masse de silice cristalline, par rapport à la masse du dioxyde de silicium employé dans le premier et/ou le second composant. Dans un mode de réalisation, le premier et/ou le second composant contient un mélange de silice cristalline et de silice pyrogénée, dans un ratio massique variant de préférence de 50/50 à 98/2, mieux de 90/10 à 97/3.

Selon encore une autre caractéristique de l'invention, le premier composant comprend au moins un retardateur de polymérisation, également nommé inhibiteur de polymérisation. Son rôle est de ralentir la vitesse de polymérisation de la composition. Il peut appartenir à la famille des cyclosiloxanes vinyliques méthyliques. Un exemple non limitatif d'un tel composé est le 2,4,6,8-tétraméthyl-2,4,6,8-tétravinylcyclotétrasiloxane (CAS 2554-06-5). Il est généralement utilisé dans une quantité massique allant de 0,005 % à 0,1 %, de préférence de 0,01 % à 0,05 %, typiquement 0,033 %, par rapport à la masse du premier composant.

Le temps de polymérisation varie généralement de 3 à 5 minutes pour une température inférieure ou égale à 23°C, et il est généralement inférieur à 3 minutes lorsque que la température est supérieure à 23°C.

Selon une autre caractéristique de l'invention, au moins l'un des premiers et deuxièmes composants comprend un pigment. L'ajout d'un pigment permet de personnaliser le troisième composant et donc le dispositif en lui-même. Différents types de pigments sont envisageables et notamment des pigments fluorescents et phosphorescents.

Selon encore une autre caractéristique de l'invention, chacun des premiers et deuxièmes composants est présent dans une quantité sensiblement égale à 3g, de manière à obtenir un troisième composant d'une quantité sensiblement égale à 6g. Une telle quantité est particulièrement adaptée à une division en deux parties sensiblement égales permettant chacune de réaliser un bouchon pour un conduit auditif, de manière à disposer d'un bouchon pour chacun des conduits auditifs gauche et droit. Il est à noter que 3 grammes de mélange est une quantité optimale pour l'insertion dans un conduit auditif sans dépassement dans la conque de l'oreille.

Selon une autre caractéristique de l'invention, l'ensemble comprend des moyens de préhension du dispositif, lesdits moyens étant allongés et destinés à être placés en partie dans le troisième composant et à s'étendre à l'extérieur du troisième composant. La mise en œuvre de moyens de préhension permet à l'utilisateur de placer ou d'ôter simplement le dispositif du conduit auditif.

Selon une autre caractéristique de l'invention, les moyens de préhension comprennent une partie de préhension s'étendant d'au moins 1 cm à l'extérieur du troisième composant et une partie de fixation destinée à être placée dans le troisième composant. La mise en œuvre de tels moyens de préhension avec deux parties permet de les fixer efficacement dans le troisième composant.

Selon une autre caractéristique de l'invention, le troisième composant est apte à être divisé en deux parties, chacune destinée à être introduite dans un conduit auditif gauche ou droit, et l'ensemble comprend des moyens d'identification gauche et droite destinés à être associés à chacune des deux parties du troisième composant. L'identification gauche ou droite du dispositif permet à l'utilisateur de pouvoir placer chaque dispositif rapidement dans le conduit auditif adéquat.

Selon encore une autre caractéristique de l'invention, les moyens d'identification gauche ou droite des deux parties du troisième composant sont intégrés dans les moyens de préhension.

L'invention porte également sur un procédé de fabrication d'au moins un dispositif d'obturation d'un conduit auditif comprenant :
- la mise en œuvre d'un premier composant comprenant de l'huile de silicone vinylique, du dioxyde de silicium et un agent de démoulage,
- la mise en œuvre d'un deuxième composant comprenant de l'huile de silicone vinylique, du dioxyde de silicium et un agent de démoulage,
- le malaxage des premier et deuxième composants jusqu'à obtenir un troisième composant homogène,
- l'introduction du troisième composant dans un conduit auditif, et
- l'attente d'au moins trois minutes avant de le retirer.

Selon une caractéristique de l'invention, le procédé comprend en outre la division du troisième composant en deux parties, et l'introduction de chaque partie du troisième composant dans un conduit auditif distinct.

Selon une autre caractéristique de l'invention, le procédé comprend aussi la mise en place d'un moyen de préhension dans chaque troisième composant.

Selon encore une autre caractéristique de l'invention, les premier et deuxième composants appartiennent à un ensemble selon l'invention.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

De plus, diverses autres caractéristiques de l'invention ressortent de la description annexée effectuée en référence aux dessins qui illustrent des formes, non limitatives, de réalisation de l'invention et où :
[Fig. 1] est une vue d'un ensemble pour fabriquer au moins un dispositif pour conduit auditif comprenant un premier et un deuxième composant.
[Fig. 2] est une vue en perspective du troisième composant homogène.

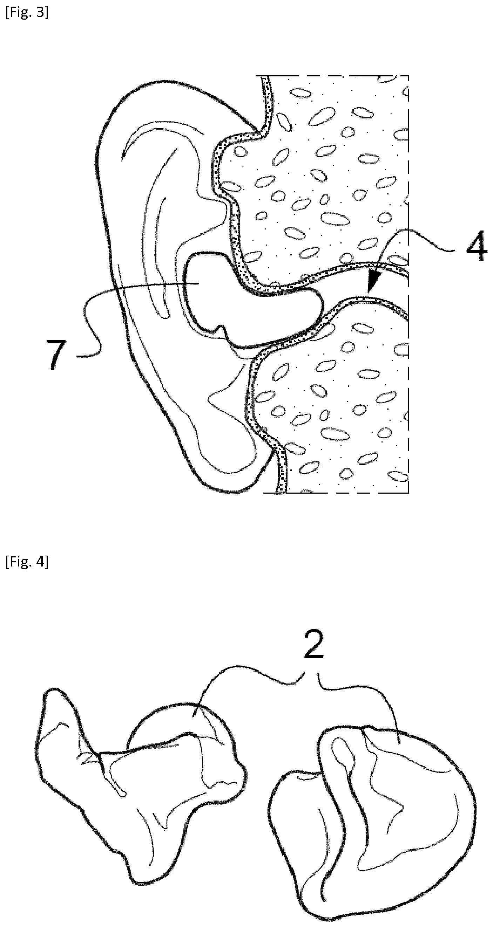

[Fig. 3] est une vue d'un dispositif obtenu à partir d'un ensemble selon l'invention placé dans un conduit auditif droit.
[Fig. 4] est une vue de deux dispositifs gauche et droit obtenus à partir d'un ensemble selon l'invention.
[Fig. 5] est une vue en perspective d'un dispositif obtenu à partir d'un ensemble selon l'invention placé dans un conduit auditif gauche.
[Fig. 6] est une vue en perspective de moyens de préhension d'un dispositif obtenu à partir d'un ensemble selon l'invention.
[Fig. 7] est une vue en perspective d'un dispositif obtenu à partir d'un ensemble selon l'invention et muni de moyens de préhension.
[Fig. 8] est une vue en perspective d'autres moyens de préhension pour deux dispositifs obtenus à partir d'un ensemble selon l'invention.
[Fig. 9] est une vue en perspective de moyens d'identification pour deux dispositifs gauche et droit obtenus à partir d'un ensemble selon l'invention.
[Fig. 10] est une vue en perspective d'un dispositif obtenu à partir d'un ensemble selon l'invention muni de moyens d'identification droits.
[Fig. 11] est une vue en perspective de deux dispositifs gauche et droit obtenus à partir d'un ensemble selon l'invention muni de moyens de préhension combinés avec des moyens d'identification droite et gauche.

Il est à noter que sur ces figures les éléments structurels et/ou fonctionnels communs aux différentes variantes peuvent présenter les mêmes références.

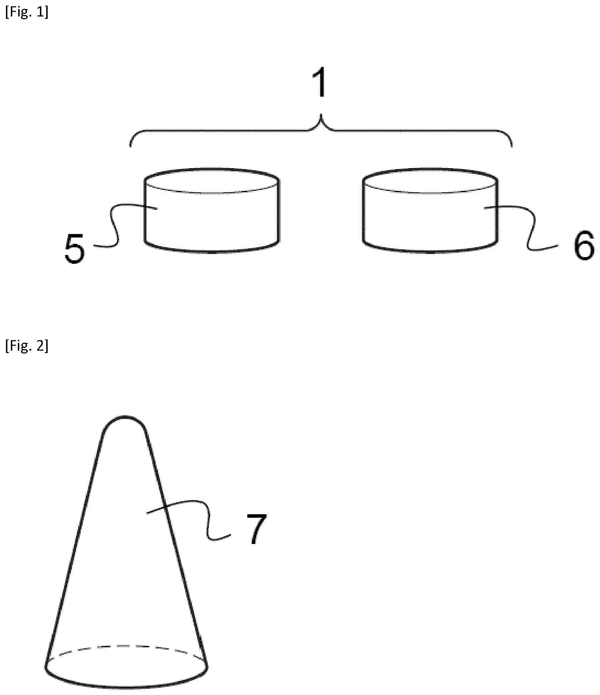

L'invention vise à proposer un ensemble 1, tel qu'illustré à la figure 1, pour fabriquer au moins un dispositif 2 destiné à être mis en œuvre dans un conduit auditif gauche 3 ou droit 4. La particularité de cet ensemble 1 est qu'il comprend deux composants 5,6 comprenant chacun de l'huile de silicone, du dioxyde de silicium et un agent de démoulage, les deux composants 5,6 étant destinés à être mélangés afin d'obtenir un troisième composant 7 homogène, illustré à la figure 2, ayant une dureté comprise entre 10 et 30 shore A.

**[Tableau 1]**

| | Premier composant 5 | Deuxième composant 6 |
|---|---|---|
| Huile de silicone vinylique | 51 | 45 |
| Dioxyde de silicium | 36 | 42 |
| Catalyseur au platine | <1 | |
| Agent de démoulage - Pigments | 12 | |
| Agent de structure - Agent de démoulage - Pigments | | 12 |
| Agent de réticulation d'huile de silicone contenant de l'hydrogène | | 1 |
| Retardateur de polymérisation | 0,033 | |

Le tableau 1 est un tableau détaillant un exemple de composition en pourcentage en masse des premier et deuxième composants 5,6 d'un ensemble 1 selon l'invention permettant d'obtenir un troisième composant 7 ayant une dureté sensiblement égale à 20 Shore A.

Selon cet exemple de réalisation, il est possible d'obtenir facilement d'autres valeurs de dureté shore A du troisième composants 7, en modifiant les concentrations de certains composés du premier composant 5, à savoir les proportions en masse d'huile de silicone vinylique ainsi que du dioxyde de silicium.

**[Tableau 2]**

| | Premier composant 5 | Deuxième composant 6 |
|---|---|---|
| Huile de silicone vinylique | 57 | 45 |
| Dioxyde de silicium | 30 | 42 |
| Catalyseur au platine | <1 | |
| Agent de démoulage - Pigments | 12 | |
| Agent de structure - Agent de démoulage - Pigments | | 12 |
| Agent de réticulation d'huile de silicone contenant de l'hydrogène | | 1 |
| Retardateur de polymérisation | 0,033 | |

Le tableau 2 est un tableau détaillant un exemple de composition en des premier et deuxième composants 5,6 d'un ensemble 1 selon l'invention permettant d'obtenir un troisième composant 7 ayant une dureté sensiblement égale à 10 Shore A.

**[Tableau 3]**

| | Premier composant 5 | Deuxième composant 6 |
|---|---|---|
| Huile de silicone vinylique | 43 | 45 |
| Dioxyde de silicium | 44 | 42 |
| Catalyseur au platine | <1 | |
| Agent de démoulage - Pigments | 12 | |
| Agent de structure - Agent de démoulage - Pigments | | 12 |
| Agent de réticulation d'huile de silicone contenant de l'hydrogène | | 1 |
| Retardateur de polymérisation | 0,033 | |

Le tableau 3 est un tableau détaillant un exemple de composition en pourcentage en masse des premier et deuxième composants 5,6 d'un ensemble 1 selon l'invention permettant d'obtenir un troisième composant 7 ayant une dureté sensiblement égale à 30 Shore A.

Selon ces trois exemples non limitatifs, l'agent de contrôle de la structure peut comprendre du décaméthylcyclopentasiloxane qui est un composant qui interagit favorablement avec l'huile de silicone. D'autres agents de contrôle peuvent être utilisés. Dans chacun des tableaux 1 à 3, le premier et le second composant contiennent chacun 1,7 % en masse de silice pyrogénée (surface spécifique de 200 ± 25 m²/g), le reste du dioxyde de silicium étant présent sous forme de quartz (surface spécifique de 125 ± 20 m²/g).

Afin de mettre en œuvre l'ensemble 1 selon l'invention, un utilisateur doit malaxer ensemble le premier composant 2 et le deuxième composant 3 jusqu'à obtenir un troisième composant 7 homogène.

S'il souhaite utiliser l'ensemble 1 pour ses deux conduits auditifs gauche 3 et droit 4, l'utilisateur doit diviser le troisième composant 7 en deux parties sensiblement égales. Chaque partie est ensuite façonnée sous une forme allongée telle qu'une forme conique illustrée à la figure 2. L'utilisateur pourrait aussi conformer en cylindre chaque partie devant être introduite dans un conduit auditif 3,4. L'utilisateur place ensuite une partie du mélange dans chacun de ses conduits auditifs 3,4 afin de les obturer. L'utilisateur peut à sa convenance lisser la partie extérieure visible dans l'oreille.

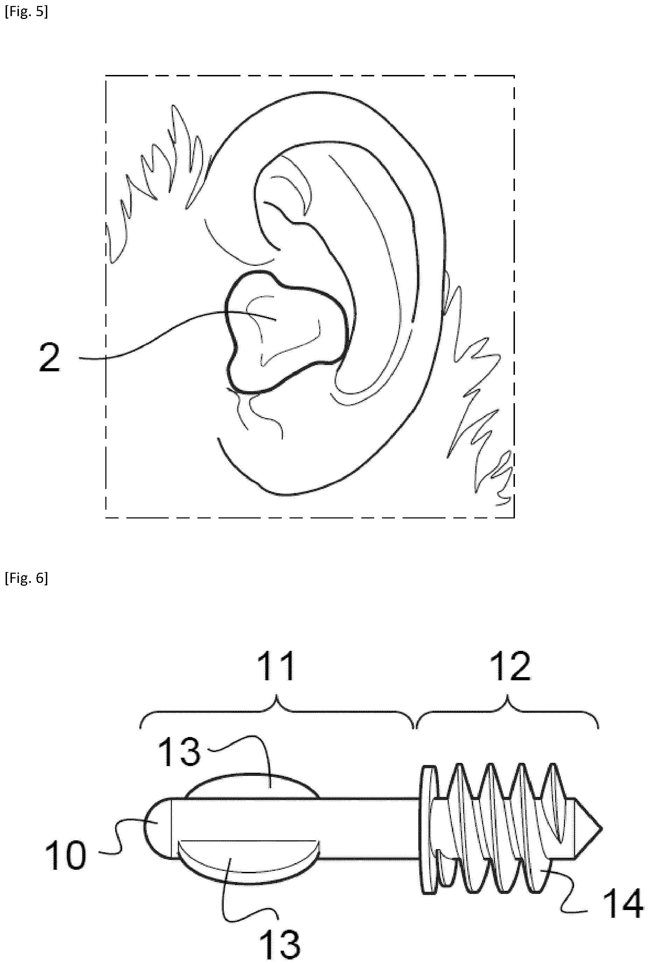

La figure 5 illustre un dispositif 2 mis en place dans un conduit auditif gauche 4, la partie visible du dispositif 2 placé dans l'oreille y a un aspect lisse et plat. Les particularités du troisième composant 7 impliquent un temps de séchage d'environ trois minutes. Ensuite, chacune des parties gauche et droite peut être retirée et les dispositif 2 gauche et droit sont alors immédiatement fonctionnels.

Selon une variante de l'invention, l'ensemble 1 comprend des moyens de préhension 10 destinés à faciliter la mise en place et le retrait du dispositif 2 pour conduit auditif 3,4.

Ainsi, la figure 6 illustre un exemple de réalisation d'un moyen de préhension 10 comprenant une partie de préhension 11 et une partie de fixation 12 destinée à être insérée dans le dispositif 2.

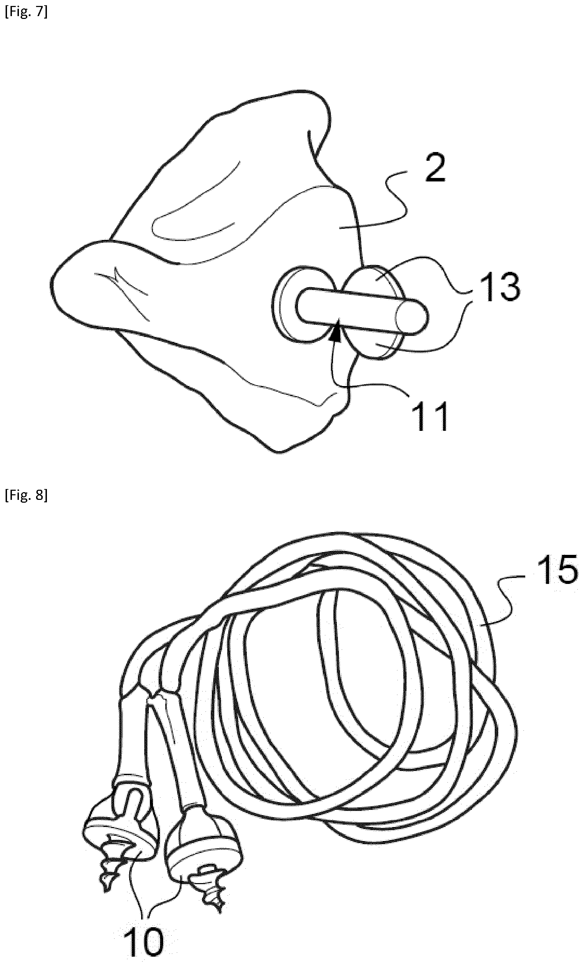

Selon cet exemple de réalisation, la partie de préhension 11 comprend deux ergots 13 destinés à faciliter une préhension entre le pouce et l'index et la partie de fixation 12 comprend une tige filetée 14 destinée à être visser dans un dispositif 2 obtenu à partir d'un ensemble 1 selon l'invention, de manière à obtenir un dispositif 2 tel qu'illustré à la figure 7.

La figure 8 illustre un autre exemple de moyens de préhension 10, où la partie de préhension 11 d'un premier moyen de préhension 10 est fixée à un cordon souple 15 attaché à son autre extrémité à la partie de préhension d'un second moyen de préhension 10. L'utilisation d'un cordon permet de lier les deux dispositifs 2 gauche et droit.

Selon ces deux exemples illustrés, les deux parties de fixation 12 sont des tiges filetées 14. D'autres moyens de fixation sont envisageables tels qu'une tige munie de dents en sapin.

Selon une autre variante de réalisation de l'invention, l'ensemble 1 comprend des moyens d'identification 20 permettant de distinguer le dispositif 2 destiné au conduit auditif droit 3 du dispositif 2 destiné au côté gauche.

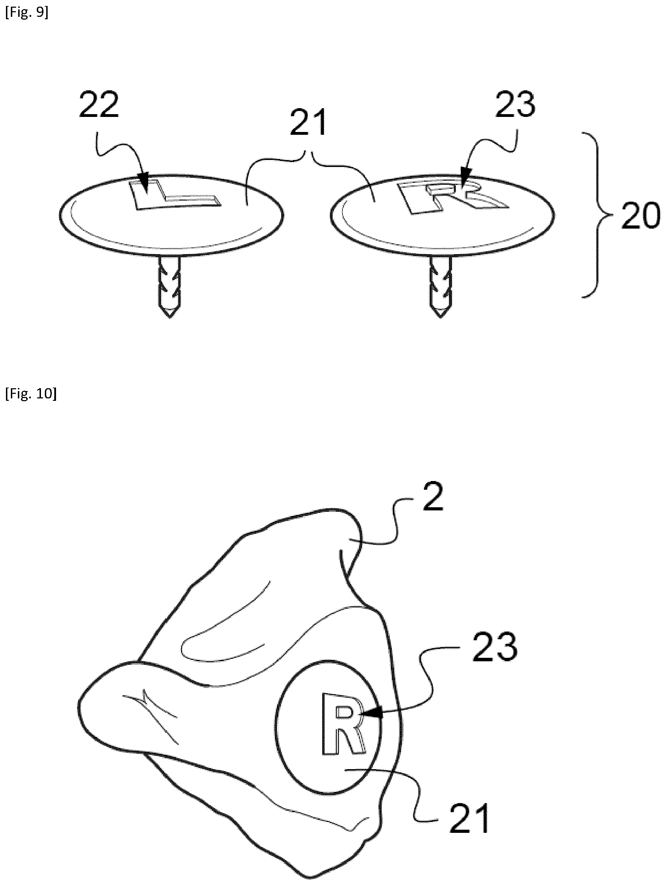

La figure 9 illustre un exemple de moyens d'identification 20 d'un dispositif 2 mis en œuvre à partir d'un ensemble 1 selon l'invention comprenant un premier disque 21 arborant un symbole L 22 pour Left (gauche en langue française) et un deuxième disque 21 un symbole R 23 pour Right (droite en langue française). Chacun de ces disques 21 est fixé à une tige affutée 24 destinée à être insérée dans chacun des dispositifs 2 gauche et droit. La figure 10 illustre un moyen d'identification 20 droit, arborant le symbole R 23, placé dans un dispositif 2 destiné à être mis en œuvre dans un conduit auditif droit 4.

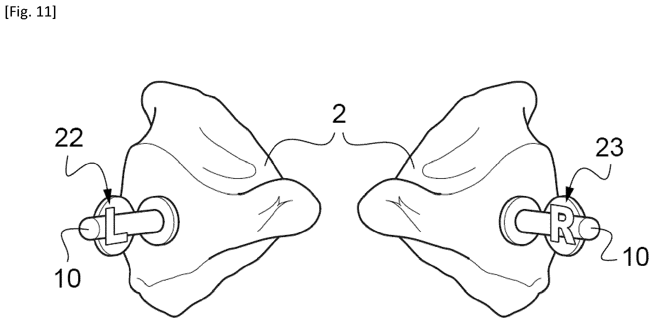

Les moyens d'identifications 20 peuvent aussi être intégrés dans les moyens de préhension 10 comme illustré à la figure 11 où le symbole R 23 est situé sur la partie de préhension 11 du dispositif 2 destiné à être mis en œuvre dans le conduit auditif droit 4 et le symbole L 22 est situé sur la partie de préhension 11 du dispositif 2 destiné à être mis en œuvre dans le conduit auditif gauche 5.

Bien entendu, diverses autres modifications peuvent être apportées à l'invention dans le cadre des revendications annexées.

## Revendications

1. Ensemble (1) pour fabriquer au moins un dispositif (2) d'obturation d'un conduit auditif (3,4), **caractérisé en ce qu'**il comprend un premier et un deuxième composant (5,6) comprenant chacun de l'huile de silicone vinylique, du dioxyde de silicium et un agent de démoulage, les deux composants (5,6) étant destinés à être mélangés ensemble pour former un troisième composant (7) homogène ayant une dureté comprise entre 10 et 30 shore A, et ledit troisième composant (7) est destiné à être introduit dans au moins un conduit auditif (3,4).

2. Ensemble (1) selon la revendication 1, **caractérisé en ce que** le premier composant (5) comprend en outre un catalyseur au platine et le deuxième composant (6) comprend en outre un agent de réticulation d'huile de silicone contenant de l'hydrogène et un agent de contrôle de la structure.

3. Ensemble (1) selon la revendication 2, **caractérisé en ce que** le premier composant (5) comprend de l'huile de silicone vinylique dans une proportion en masse comprise entre 43 et 57%, du dioxyde de silicium dans une proportion en masse comprise entre 30 et 44%, un catalyseur au platine dans une proportion en masse de moins de 1% et un agent de démoulage dans une proportion en masse d'environ 12%, et le deuxième composant (6) comprend de l'huile de silicone dans une proportion en masse d'environ 45%, du dioxyde de silicium dans une proportion en masse d'environ 42%, un agent de réticulation d'huile de silicone contenant de l'hydrogène dans une proportion en masse d'environ 1%, un agent de contrôle de la structure et un agent de démoulage dans une proportion en masse d'environ 12%.

4. Ensemble (1) selon la revendication 3, **caractérisé en ce que** le premier composant (5) comprend de l'huile de silicone vinylique dans une proportion en masse d'environ 51% et du dioxyde de silicium dans une proportion en masse d'environ 36%.

5. Ensemble (1) selon l'une des revendications 1 à 4, **caractérisé en ce qu'**au moins l'un des premier et deuxième composants (5,6) comprend un pigment.

6. Ensemble (1) selon l'une des revendications 1 à 5, **caractérisé en ce que** chacun des premier et deuxième composants (5,6) est présent dans une quantité sensiblement égale à 3g, de manière à obtenir un troisième composant (7) d'une quantité sensiblement égale à 6g.

7. Ensemble (1) selon l'une des revendications 1 à 6, **caractérisé en ce qu'**il comprend des moyens de préhension (10) du dispositif (2), lesdits moyens (10) étant allongés et destinés à être placés en partie dans le troisième composant (7) et destinés à s'étendre à l'extérieur du troisième composant (7).

8. Ensemble (1) selon la revendication 7, **caractérisé en ce que** les moyens de préhension (10) comprennent une partie de préhension (11) s'étendant d'au moins 1 cm à l'extérieur du troisième composant (7) et une partie de fixation (12) destinée à être placée dans le troisième composant (7).

9. Ensemble (1) selon l'une des revendication 1 à 8, **caractérisé en ce que** le troisième composant (7) est apte à être divisé en deux parties, chacune destinée à être introduite dans un conduit auditif gauche (3) ou droit (4), et **en ce que** l'ensemble (1) comprenne des moyens d'identification (20) gauche et droite destinés à être associés à chacune des deux parties du troisième composant (7).

10. Ensemble (1) selon la revendication 9, **caractérisé en ce que** les moyens d'identification (20) gauche ou droite des deux parties du troisième composant (7) sont intégrés dans les moyens de préhension (10).

11. Procédé (25) de fabrication d'au moins un dispositif (2) d'obturation d'un conduit auditif (3,4), **caractérisé en ce qu'**il comprend :
- la mise en œuvre d'un premier composant (5) comprenant de l'huile de silicone vinylique, du dioxyde de silicium et un agent de démoulage,
- la mise en œuvre d'un deuxième composant (6) comprenant de l'huile de silicone, du dioxyde de silicium et un agent de démoulage,
- le malaxage des premier et deuxième composants (5,6) jusqu'à obtenir un troisième composant (7) homogène,
- l'introduction du troisième composant (7) dans un conduit auditif (3,4), et
- l'attente d'au moins trois minutes avant de le retirer.

12. Procédé (25) selon la revendication 11, **caractérisé en ce qu'**il comprend en outre la division du troisième composant (7) en deux parties, et l'introduction de chaque partie du troisième composant (7) dans un conduit auditif distinct (3,4).

13. Procédé (25) selon l'une des revendications 11 ou 12, **caractérisé en ce qu'**il comprend aussi la mise en place d'un moyen de préhension (10) dans chaque troisième composant (7).

14. Procédé (25) selon l'une des revendications 11 à 13, **caractérisé en ce que** le premier composant (5) comprend de l'huile de silicone vinylique dans une proportion en masse comprise entre 43 et 57%, du dioxyde de silicium dans une proportion en masse comprise entre 30 et 44%, un catalyseur au platine dans une proportion en masse de moins de 1% et un agent de démoulage dans une proportion en masse d'environ 12%, et le deuxième composant (6) comprend de l'huile de silicone dans une proportion en masse d'environ 45%, du dioxyde de silicium dans une proportion en masse d'environ 42%, un agent de réticulation d'huile de silicone contenant de l'hydrogène dans une proportion en masse d'environ 1%, un agent de contrôle de la structure et un agent de démoulage dans une proportion en masse d'environ 12%.

15. Procédé (25) selon l'une des revendications 11 à 14, **caractérisé en ce que** les premier et deuxième composants (5,6) appartiennent à un ensemble (1) selon l'une des revendications 1 à 10.
